# EUROPÄISCHE PATENTSCHRIFT

(11) **EP 1 954 350 B1**
(45) Veröffentlichungstag und Bekanntmachung des Hinweises auf die Patenterteilung: **28.09.2011**
(21) Anmeldenummer: 06818557.8
(22) Anmeldetag: 15.11.2006
(51) Int. Cl.: A61N 1/39

(54) **AUTOMATISCHE EXTERNE DEFIBRILLATORVORRICHTUNG**
EXTERNAL AUTOMATIC DEFIBRILLATOR
DEFIBRILLATEUR EXTERNE AUTOMATIQUE

(30) Priorität: 15.11.2005 DE 102005054778
(43) Veröffentlichungstag der Anmeldung: 13.08.2008
(73) Patentinhaber: Metrax GmbH, D-78628 Rottweil (DE)
(72) Erfinder: BUCHER, Heinz, 78615 Rottweil (DE); STORK, Wilhelm, 76831 Impflingen (DE); KRAFT, Norbert, 79618 Rheinfelden (DE)
(74) Vertreter: Fleck, Hermann-Josef
(86) Internationale Anmeldenummer: PCT/EP2006/010959
(87) Internationale Veröffentlichungsnummer: WO 2007/057169

(56) Entgegenhaltungen:
- WO-A-2004/054656
- US-A- 4 088 138
- US-A- 4 610 254
- US-A- 5 342 404
- US-A1- 2002 103 508
- US-A1- 2005 085 738
- US-A1- 2005 131 465

## Beschreibung

Die Erfindung bezieht sich auf eine automatische externe Defibrillatorvorrichtung mit einer Anbringvorrichtung zum äußeren Anlegen und Tragen derselben an einem Patienten, mit einer Erkennungseinrichtung zum Erfassen eines mittels elektrischem Schock therapierbaren abnormalen Ereignisses der Herztätigkeit und mit einer Defibrillationseinrichtung zum Beaufschlagen des Patienten mit einem Schock nach Erfassen des abnormalen Ereignisses.

Eine automatische externe Defibrillatorvorrichtung mit einer Anbringvorrichtung zum äußeren Anlegen und Tragen an einem Patienten ist in der US 2005/131465 A1 angegeben. Bei dieser bekannten Defibrillatorvorrichtung ist eine Erfassungseinrichtung für den Aktivitätszustand eines Patienten vorgesehen, deren Daten einer Entscheidung zugrunde gelegt werden, ob eine Defibrillation vorgenommen wird oder nicht. Auch werden zeitliche Ablaufsteuerungen zum Einleiten einer Defibrillation vorgenommen.

Weitere Defibrillatorvorrichtungen zeigen die US 4,088,138 A, die US 4,610,254 A, US 2005/085738 A1, US 5,342,404 A und die WO 2004/054656 A.

Eine weitere automatische externe Defibrillatorvorrichtung ist in der DE 689 27 898 T2 angegeben. Bei dieser bekannten Defibrillatorvorrichtung ist eine Anbringvorrichtung mit einem Oberkörpergurtzeug oder einer Tragekleidung vorgesehen, mit dem eine Elektrodeneinrichtung am Körper des Patienten anbringbar ist. Auch ist eine Überwachungseinrichtung vorgesehen, die wenigstens eine Überwachungseinheit auf dem Gurtzeug umfasst, wobei auch eine Brustbewegung beim Atmen erfasst werden kann. Eine Schwierigkeit bei derartigen tragbaren automatischen externen Defibrillatorvorrichtungen besteht darin, immer genügend elektrische Energie für die Schockbehandlung sicherzustellen.

Ein ähnlicher tragbarer Defibrillator, mit dem die Herztätigkeit analysiert und festgestellt wird, ob eine Defibrillation erforderlich ist, ist auch in der US 4,576,170 dargestellt.

Eine weitere tragbare externe Defibrillatorvorrichtung ist in der US 2003/0004547 A1 gezeigt. Hierbei geht es insbesondere um die Ausbildung der dabei vorgesehenen Elektroden für eine lange Tragdauer.

Der Erfindung liegt die Aufgabe zugrunde, eine automatische externe Defibrillatorvorrichtung der vorstehend genannten Art bereitzustellen, bei der eine erhöhte Funktionssicherheit erreicht wird.

Diese Aufgabe wird mit den Merkmalen des Anspruches 1 gelöst.

Gemäss der Erfindung ist ein Zeitmesser vorhanden der nach Erkennen eines ventrikulären Flimmerns für eine vorbestimmte Zeitspanne von höchstens 90 Sekunden oder einer Minute gestartet wird, wobei die Erkennungseinrichtung zum Erfassen des abnormalen Ereignisses innerhalb der Zeitspanne ausgebildet ist, während der Zeitspanne die Bewegungsanalyse durchgeführt wird und nach Ablauf der Zeitspanne zur Vorbereitung der Schockbeaufschlagung übergegangen wird.

Die kurz bemessene Zeitspanne zum Erfassen des abnormalen Ereignisses basiert auf einer schnellen Erkennung und gewährleistet eine schnelle Entscheidung, wenn ein Defibrillationsimpuls bzw. -impulsfolge erforderlich und abzugeben ist. Es hat sich gezeigt, dass umso weniger Energie für den Schock benötigt wird, je kürzer die Zeit von einem Kammerflimmern zum Schock ist. Einerseits ist die relativ geringe Energie beim Schock für das Herz schonender und andererseits muss nicht so viel Energie in der Energieversorgung, insbesondere dem Akkumulator vorgehalten werden, um eine ausreichende Schockenergie sicher zu stellen. Vorteilhaft ist hierbei, wenn die Vorteilhafte Ausgestaltungen bestehen dabei darin, dass die Zusatz-Sensoreinrichtung mindestens einen Bewegungssensor und/oder mindestens einen Lagesensor aufweist, mit dem oder denen eine Bewegung bzw. die Lage des Körpers feststellbar ist. Wird hierbei beispielsweise festgestellt, dass der Patient sich bewegt, kann die Erkennung eines abnormalen Ereignisses beispielsweise über das EKG-Signal als fehlerhaft verworfen und eine Neumessung vorgenommen werden. Auch kann festgestellt werden, wenn eine weitere Person durch äußeren Eingriff den Körper des Patienten bewegt und dadurch selbst vom Schock gefährdet würde. Steht beispielsweise der Oberkörper des Patienten aufrecht, könnte die Erfassung eines abnormalen Ereignisses fehlerhaft sein oder der Patient könnte nach einem Schock umfallen und sich verletzen. Durch die Zusatzinformationen werden bessere Unterscheidungen und Behandlungsmaßnahmen in solchen Situationen erreicht.

Dabei bestehen vorteilhafte Ausgestaltungsvarianten darin, dass der Bewegungssensor und/oder der Lagesensor mindestens einen Beschleunigungssensor aufweist/aufweisen.

Ist vorgesehen, dass die Zusatz-Sensoreinrichtung eine Dehnmesseinrichtung aufweist, können z.B. Atembewegungen mit einem entsprechenden Algorithmus sicher erkannt und in die Bewertung zum Erkennen eines abnormalen Ereignisses einbezogen werden.

Robuste Erkennungskriterien für die Entscheidung, ob ein Schock ausgelöst werden soll, werden ferner dadurch erhalten, dass die Zusatz-Sensoreinrichtung Sensoren zum Erfassen des neurologischen Zustandes des Patienten, Sensoren zum Erfassen des Lidschlagreflexes und/oder Sensoren zum Erfassen der Augenbewegung aufweist, weiterhin dadurch, dass eine Sprachausgabe- und/oder Spracheingabeeinheit für Sprachinformation an oder von einer entfernten Kontrollstation vorhanden ist und dass die Erkennungseinrichtung zum Erfassen und Bewerten von Bewegungen oder Bewegungsmustern im Anschluss an eine Bewegungsaufforderung an den Patienten ausgebildet ist und/oder zur Bewertung von Sprechsignalen des Patienten, ferner dadurch, dass die Erkennungseinrichtung dazu ausgebildet ist, auf der Grundlage definierter durch den Patienten eingenommener Lage- und/oder Bewegungszustände eine Kalibrierung durchzuführen, und auch dadurch, dass die Erkennungseinrichtung so ausgebildet ist, dass die Schockauslösung bei bestimmten Lagen des Patienten priorisiert oder nur bei bestimmten Lagen auslösbar ist.

Die Erfindung wird nachfolgend anhand von Ausführungsbeispielen unter Bezugnahme auf die Zeichnung erläutert. Es zeigen:
- Fig. 1: eine schematische Darstellung einer Defibrillatorvorrichtung und
- Fig. 2: ein Ausführungsbeispiel für eine Analyse- und Ablaufsteuerung.

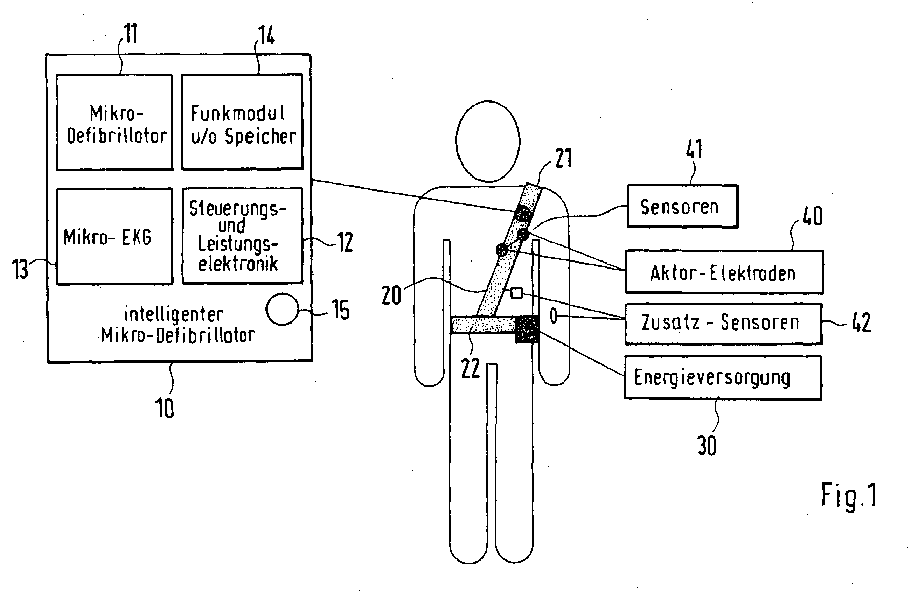

Die Fig. 1 zeigt eine automatische externe Defibrillatorvorrichtung, die dauerhaft am Körper eines von einem Herztod gefährdeten Patienten anlegbar und tragbar ist, bis das Risiko unter eine bestimmte Schwelle sinkt.

Die mittels einer Tragevorrichtung 20 am Körper des Patienten gehaltene mobile Defibrillatorvorrichtung weist einen Elektronikteil 10, einen Defibrillatorteil 11, einen Ansteuerteil 12, einen Überwachungsteil 13 und einen Registrierteil 14 auf.

Die Tragevorrichtung 20 weist beispielsweise mindestens einen Schulterträger 21 und mindestens einen Gurt 22 in Form eines Brust- und/oder Hüftgurtes auf. An der Tragevorrichtung 20 ist auch eine Energieversorgung 30 für den Elektronikteil 10 gehalten, wobei der Aufbau auch so ausgeführt sein kann, dass der Elektronikteil 10 gemeinsam in einem Gehäuse mit der Energieversorgung 30 untergebracht ist. Außerdem werden von der Tragevorrichtung 20 auch Aktor-Elektroden 40, insbesondere die Patientenelektroden, sowie gegebenenfalls weiterhin Sensoren einer Zusatz-Sensoreinrichtung 42 gehalten. Die Patientenelektroden können dabei gleichzeitig auch Sensorelemente 41 beinhalten, um z.B. Impedanzmessungen durchzuführen, und auch EKG-Signale können mit geeigneten Elektroden, die ebenfalls in die Patientenelektroden integriert sein können, erfasst und mit dem Elektronikteil 10 ausgewertet werden, wie an sich bekannt.

Der Elektronikteil 10 weist neben einer Steuerungs- und Leistungselektronik insbesondere für den Defibrillatorteil 11 auch einen Schaltungsteil mit einer Erkennungseinrichtung zum Erfassen eines abnormalen Ereignisses der Herztätigkeit des Patienten auf, wobei vorteilhaft eine Rechnereinheit mit geeigneten Algorithmen und zugeordneten Speichereinrichtungen vorhanden ist. Die Speichereinrichtung ist Teil des Registrierteils 14, der weiterhin mit einem Funkmodul zum Senden und Empfangen von Patientendaten bzw. in dem Elektronikteil 10 aufbereiteter Daten an eine entfernte Kontrollstation ausgebildet sein kann. Die Erkennungseinrichtung weist des Weiteren eine Bewertungseinrichtung auf, um aus die Herztätigkeit des Patienten betreffenden Patientendaten und gegebenenfalls mittels einer Zusatz-Sensoreinrichtung erfasster Zusatzinformationen ein abnormales Ereignis der Herztätigkeit durch Vergleich mit Solldaten zu erkennen. Die Solldaten können von vornherein abgestimmt auf den jeweiligen Patienten eingegeben sein und/oder auch entsprechend einer Vorgeschichte des Ereignisses ermittelt, gegebenenfalls angepasst und geeignet vorgegeben werden. Die Patientendaten werden über entsprechende Sensoren gewonnen und über Anschlusselemente der Erkennungseinrichtung zugeführt und bei Bedarf wird von dieser ein Erkennungssignal an die Defibrillationseinrichtung abgegeben, um bei Erfordernis automatisch einen Schock auszulösen. Auch können vorbekannte Patientendaten, die besondere, individuelle Eigenschaften eines Patienten betreffen, in dem Elektronikteil 10, insbesondere der Erkennungseinrichtung gespeichert sein, um sie zur Erkennung und Bewertung eines abnormalen Ereignisses der Herztätigkeit einbeziehen und bei der Entscheidung über die Auslösung eines Defibrillationsschocks einzubeziehen.

Die Zusatzinformationen können beispielsweise Bewegungsdaten oder Lagedaten des Patientenkörpers oder eine Atmungstätigkeit des Patienten oder Kombinationen daraus oder weiterer Daten enthalten, so dass die Erkennungssicherheit des abnormalen Ereignisses z.B. durch Feststellen bestimmter Bewegungen oder Bewegungsmuster erhöht werden kann oder die Abgabe des Erkennungssignals an die Defibrillationseinrichtung unterbunden bzw. eine Defibrillation in einem ungünstigen Zustand vermieden werden kann. Die Zusatz-Sensoreinrichtung 42 weist beispielsweise mindestens einen Bewegungssensor und/oder mindestens einen Lagesensor auf, die vorteilhaft als Beschleunigungssensor, insbesondere 3D-Beschleunigungssensor ausgebildet sind, die vorteilhaft an der Hüfte oder vor der Brust angebracht sind. Zum Erfassen einer Atemtätigkeit eignen sich z.B. ein dehnbares Band mit Veränderung der Leitfähigkeit und ein entsprechender Auswertealgorithmus.

Bei Verwendung mehrerer Bewegungssensoren kann ein genaueres Bild der Körperaktivität gewonnen und damit besser zwischen aktiver und passiver Bewegung des Patienten unterschieden werden. Dies kann z.B. durch Auswertung eines Differenzsignals geschehen, durch die als Gleichtaktsignal einwirkende Bewegungskomponenten von außen als passive Bewegung erkannt werden können. Zum anderen kann eine Platzierung der Sensoren z.B. in der entsprechend ausgebildeten Tragevorrichtung 20 in der Nähe der Extremitäten dazu genutzt werden, Bewegungen z.B. im Sitzen besser zu erkennen.

Bei einer vorteilhaften Ausgestaltung der Defibrillatorvorrichtung ist eine Sprachausgabe- und/oder Spracheingabe für Sprachinformationen von oder zu einer entfernten Kontrollstation vorgesehen. Dadurch hat das System mit der Defibrillatorvorrichtung die Möglichkeit, bei unklarer Signalauswertung den Patienten zu bestimmten Bewegungen und/oder Körperlageänderungen aufzufordern. Registriert das System in der Folge eine Bewegung oder bestimmte charakteristische Bewegungsmuster, die mittels der Erkennungseinrichtung z.B. durch Vergleich mit gespeicherten Bewegungen bzw. Bewegungsmustern erkannt werden, kann auf einen Patienten bei Bewusstsein und Körperaktivität geschlossen werden und ein Schock verhindert werden oder eine geeignete Situation abgewartet werden. Bei dieser Ausgestaltung besteht eine weitere Ausbildung darin, dass über die Spracheingabe der Patient zur Einnahme bestimmter Körperlagen aufgefordert wird und bei diesen bestimmten Körperlagen eine Kalibrierung des Systems, insbesondere der Erkennungseinrichtung, durchgeführt wird. Dabei besteht eine weitere Ausbildung darin, dass der Patient vor Einnehmen bestimmter Körperlagen, die eine Schockindikation begünstigen (z.B. Liegen während des Schlafs), dies der Defibrillatorvorrichtung über eine Eingabeeinheit mitteilt, wodurch die Signalauswertung modifizierbar ist, z.B. durch Änderung von Gewichtsfaktoren bei der rechnerischen Auswertung der Signale.

Bei einer weiteren Ausbildung ist vorgesehen, dass der Patient die Schockabgabe durch Drücken von Bedientasten aktiv unterdrücken kann. Die Vorbereitung eines Schocks kann durch verschiedene Signale (optisch, akustisch, taktil) angekündigt werden.

Eine weitere Ausgestaltung besteht darin, dass Zusatzsensoren vorgesehen sind, mit denen Rückschlüsse auf den neurologischen Zustand des Patienten möglich sind. Beispielsweise sind die weiteren Zusatzsensoren zur Generierung von evozierten Potenzialen ausgebildet, die über dafür ausgebildete Signalquellen hervorgerufen und über Sensorelemente detektiert werden. Eine weitere Ausführungsvariante besteht in der Überprüfung des Lidschlagreflexes und/oder der Augenbewegung durch weitere Zusatzsensoren, wobei eine Ausgestaltung in der Aufnahme eines Elektrookulogramms bestehen kann.

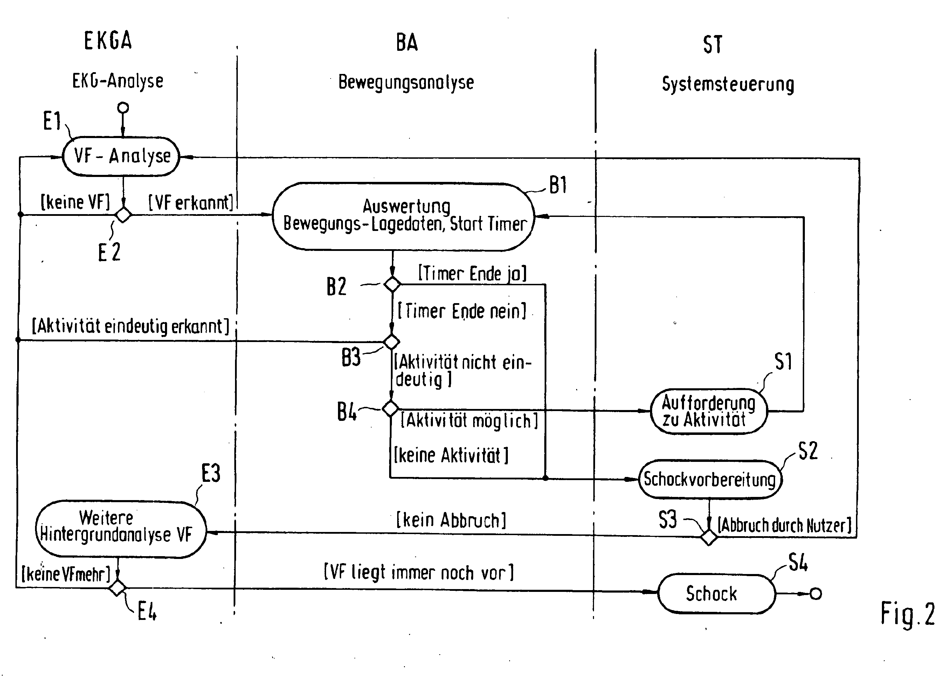

In Fig. 2 ist ein Beispiel für eine Analyse- und Ablaufsteuerung für die Überprüfung, ob ein Schock ausgelöst werden soll, und zur gegebenenfalls erforderlichen Auslösung eines Schocks angegeben. Die Steuerung ist anhand von Aktivitätsdiagrammen dargestellt, unterteilt in einen Bereich einer EKG-Analyse EKGA, einer Bewegungsanalyse BA und einer Systemsteuerung ST.

Bei der EKG-Analyse EKGA wird ausgehend von einem Startpunkt in einem Schritt E1 überprüft, ob ein ventrikuläres Flimmern (VF) vorliegt. Wird in einem Schritt E2 entschieden, dass kein ventrikuläres Flimmern vorliegt, so wird zu dem Schritt E1 der Analyse des ventrikulären Flimmerns zurückgekehrt. Wird in dem Schritt E2 entschieden, dass ventrikuläres Flimmern erkannt ist, wird in den Bereich der Bewegungsanalyse BA in einem Schritt B1 eine Auswertung der Bewegungs-/Lagedaten vorgenommen und ein Zeitmesser (Timer) für eine vorbestimmte Zeitdauer gestartet. Wird in einem anschließenden Schritt B2 der Bewegungsanalyse BA das Ende der Zeitdauer festgestellt, wird zu einer Schockvorbereitung in einem Schritt S2 im Bereich der Systemsteuerung ST übergegangen. Wird in dem Schritt B2 festgestellt, dass die Zeitdauer noch nicht abgelaufen ist, wird in einem anschließenden Schritt B3 im Bereich der Bewegungsanalyse BA überprüft, ob eine Aktivität des Patienten erkannt wurde, wozu der Patient zuvor im Bereich der Systemsteuerung in einem Schritt S1 z.B. akustisch (z.B. über die oben genannte Spracheingabe), optisch oder taktil aufgefordert worden ist. Wurde eine Aktivität im Schritt B3 eindeutig erkannt, wird zu dem Schritt E1 der ventrikulären Flimmeranalyse zurückgekehrt. Wird in dem Schritt B3 eine Aktivität nicht eindeutig erkannt, wird in einem weiteren Schritt B4 der Bewegungsanalyse festgestellt, ob eine Aktivität möglich ist oder nicht. Ist eine Aktivität möglich, wird zu dem Schritt S1 der Aufforderung zur Aktivität im Bereich der Systemsteuerung ST übergegangen. Wird in dem Schritt B4 festgestellt, dass keine Aktivität erkennbar bzw. möglich ist, wird zu dem Schritt S2 der Schockvorbereitung im Bereich der Systemsteuerung ST übergegangen. Wird in einem anschließenden Schritt S3 im Bereich der Systemsteuerung ST festgestellt, dass der Benutzer die Schockvorbereitung z.B. durch Eingabe einer betreffenden ersten Information (s. oben) abgebrochen hat, wird zu dem Schritt E1 der ventrikulären Flimmeranalyse im Bereich der EKG-Analyse EKGA zurückgegangen. Wird in dem Schritt S3 kein Abbruch festgestellt, wird zu einem Schritt E3 einer weiteren Hintergrundanalyse des ventrikulären Flimmerns im Bereich der EKG-Analyse EKGA übergegangen. Wird in einem daran anschließenden Schritt E4 festgestellt, dass kein ventrikuläres Flimmern mehr vorliegt, wird zu dem Schritt E1 der ventrikulären Flimmeranalyse zurückgegangen. Wird in dem Schritt E4 festgestellt, dass immer noch ein ventrikuläres Flimmern vorliegt, wird in einem Schritt S4 im Bereich der Systemsteuerung ST ein Schock ausgelöst. Danach wird zum Endpunkt übergegangen, wonach weitere Aktivitäten, wie Analysen und Überprüfungen vorgenommen werden können.

Mit anderen Worten, der Ablauf einer Analyse unter Verbindung einer EKG- und einer Bewegungsanalyse entsprechend den Aktivitätsdiagrammen nach Fig. 2 lässt sich wie folgt darstellen:

### a) Aufforderung zu Aktivität

Der Patient wird z.B. optisch, akustisch oder taktil zu einer körperlichen Aktivität aufgefordert, wobei z.B. ein vorher spezifiziertes Bewegungsmuster zugrunde gelegt werden kann. Die eingehende Transition besteht dann darin, dass von dem betreffenden Entscheidungsknoten B4 unter der Bedingung, dass eine Aktivität möglich ist, zu dem Schritt S1 der Aufforderung zur Aktivität übergegangen wird. Die ausgehende Transition besteht darin, dass von dem Schritt S1 zu dem Schritt B1 der Auswertung der Bewegungs-/Lagedaten und Start des Zeitgebers übergegangen wird.

### b) Auswertung Bewegungs-/Lagedaten, Start Timer

Für den Fall, dass es zu einer Aufforderung an den Patienten kommt, körperliche Aktivität zu zeigen, wird der Zeitgeber für eine bestimmte Zeitdauer (Zeitfenster) gestartet, in dem dann die Aktivität stattfinden muss. Eingehende Transitionen sind hierbei, dass von dem Entscheidungsknoten E2 zu der Auswertung Bewegungs-/Lagedaten, Start Timer im Schritt B1 übergegangen wird, unter der Bedingung, dass ein ventrikuläres Flimmern erkannt wurde. Außerdem wird von dem Schritt der Aufforderung zur Aktivität übergegangen zu dem Schritt B1 der Auswertung der Bewegungs-/Ladedaten und des Starts des Timers. Ausgehende Transition ist hierbei der Übergang von Schritt B1 zu dem Entscheidungsknoten B2.

### c) Schock

Hierbei erfolgt eine Schockabgabe, danach startet die Analyse wieder neu. Eine eingehende Transition ist hierbei der Übergang von dem Entscheidungsknoten E4 zu dem Schritt S4 Schock unter der Bedingung, dass ein ventrikuläres Flimmern immer noch vorliegt. Eine ausgehende Transition besteht in dem Übergang von dem Schritt S4 Schock zu dem Endpunkt.

### d) Schockvorbereitung

Zur Schockvorbereitung gehören die Benachrichtigung des Patienten und der Umwelt durch optische, akustische und/oder taktile Signale, ein Aufladen der Kondensatoreinheit des Defibrillators und ein Austreiben des Gels in den Defibrillationselektroden. Eingehende Transitionen sind hierbei ein Übergang von dem Entscheidungsknoten B4 zur Schockvorbereitung gemäß Schritt S2 unter der Bedingung, dass keine Aktivität festgestellt wird und von dem Entscheidungsknoten B2 zur Schockvorbereitung im Schritt S2 unter der Bedingung, dass die Zeitdauer beendet ist. Eine ausgehende Transition besteht in dem Übergang von der Schockvorbereitung im Schritt S2 zu dem Entscheidungsknoten S3.

### e) VF-Analyse

Hierbei wird eine EKG-Analyse mit dem Ziel vorgenommen, ventrikuläres Flimmern zu detektieren. Eingehende Transitionen sind hierbei der Übergang vom Startpunkt zu der VF-Analyse im Schritt E1, der Übergang von dem Entscheidungsknoten E2 zurück zur VF-Analyse unter der Bedingung, dass kein ventrikuläres Flimmern erkannt wurde, der Übergang von dem Entscheidungsknoten B3 zur VF-Analyse E1 unter der Bedingung, dass eine Aktivität des Patienten eindeutig erkannt wurde, der Übergang von dem Entscheidungsknoten S3 zu der VF-Analyse E1 unter der Bedingung, dass ein Abbruch durch den Benutzer vorliegt und der Übergang von dem Entscheidungsknoten E4 zur VF-Analyse E1 unter der Bedingung, dass kein ventrikuläres Flimmern mehr vorliegt. Eine ausgehende Transition besteht darin, dass von dem Schritt E1 der VF-Analyse zu dem Entscheidungsknoten E2 übergegangen wird.

### f) Weitere Hintergrundanalyse VF

Die VF-Hintergrundanalyse im Schritt E3 läuft permanent weiter, auch wenn Bewegungsdaten usw. ausgewertet werden. Diese Analyse dient der dauerhaften Absicherung, dass weiter ein schockpflichtiger Rhythmus vorliegt. Eingehende Transition ist hierbei der Übergang von dem Entscheidungsknoten S3 zu der weiteren Hintergrundanalyse gemäß Schritt E3 unter der Bedingung, dass kein Abbruch durch den Patienten vorliegt. Ausgehende Transition ist hierbei der Übergang von der weiteren Hintergrundanalyse E3 zu dem Entscheidungsknoten E4.

Eine besondere Ausbildung der Defibrillatorvorrichtung liegt darin, dass die Erkennungseinrichtung zum schnellen Bewerten und Erkennen eines abnormalen Ereignisses der Herztätigkeit ausgebildet ist und die Defibrillationseinrichtung zur entsprechend schnellen Reaktion und Abgabe eines Defibrillationspulses ausgebildet ist. Für das schnelle und sichere Bewerten und Erkennen dienen die vorstehend genannten Maßnahmen. Insbesondere liegt die Erkennung innerhalb einer Zeitspanne, die kürzer als 2 Minuten, vorzugsweise unter 90 Sekunden oder noch besser unter 1 Minute liegt. Es hat sich nämlich herausgestellt, dass umso weniger Energie für den Schock benötigt wird, je kürzer die Zeit vom Auftreten des Kammerflimmerns bis zum Schock ist. Dies ist schonender für den Patienten und benötigt andererseits nicht so viel Energie, so dass die Energieversorgung über längere Zeit eine ausreichende Schockenergie sicherstellen kann.

## Patentansprüche

1. Automatische externe Defibrillatorvorrichtung mit einer Anbringvorrichtung zum äußeren Anlegen und Tragen derselben an einem Patienten, mit einer Erkennungseinrichtung, die zum Erfassen eines mittels elektrischem Schock therapierbaren abnormalen Ereignisses der Herztätigkeit und Analyse von Bewegungen des Patienten ausgebildet ist, mit einer Defibrillationseinrichtung zum Beaufschlagen des Patienten mit einem Schock nach Erfassen des abnormalen Ereignisses, wobei von der Erkennungseinrichtung ein Erkennungssignal an die Defibrillationseinrichtung abgebbar und von dieser der Schock automatisch abgebbar ist, und mit einer Zusatz-Sensoreinrichtung, die mindestens einen Bewegungssensor aufweist, mittels dessen eine Bewegung des Körpers des Patienten feststellbar ist, die mittels der Erkennungseinrichtung erkennbar ist, wobei ein Zeitmesser vorhanden ist, der nach Erkennen eines ventrikulären Flimmerns für eine vorbestimmte Zeitspanne von höchstens 90 Sekunden oder einer Minute gestartet wird, wobei die Erkennungseinrichtung zum Erfassen des abnormalen Ereignisses innerhalb der Zeitspanne ausgebildet ist, während der Zeitspanne die Bewegungsanalyse durchgeführt wird nach Ablauf der Zeitspanne zur Vorbereitung der Schockbeaufschlagung übergegangen wird wenn keine Bewegungen zu denen der Patient zuvor aufgefordert worden ist, eindeutig erkannt wurden.
**dadurch gekennzeichnet,**
**dass** in der Defibrillatorvorrichtung Bewegungen gespeichert sind, mit denen bestimmte Bewegungen des Patienten verglichen werden, zu denen dieser zuvor akustisch, optisch oder taktil aufgefordert wird, und dass ein Zeitmesser vorhanden ist, der nach Erkennen eines ventrikulären Flimmerns für eine vorbestimmte Zeitspanne von höchstens 90 Sekunden oder einer Minute gestartet wird, wobei die Erkennungseinrichtung zum Erfassen des abnormalen Ereignisses innerhalb der Zeitspanne ausgebildet ist, während der Zeitspanne die Bewegungsanalyse durchgeführt wird nach Ablauf der Zeitspanne zur Vorbereitung der Schockbeaufschlagung übergegangen wird wenn keine Bewegungen zu denen der Patient zuvor aufgefordert worden ist, eindeutig erkannt wurden.

2. Defibrillatorvorrichtung nach Anspruch 1
wobei
bei der Bewegungsanalyse zwischen passiver und aktiver Bewegung unterschieden wird.

3. Defibrillatorvorrichtung nach einem der vorhergehenden Ansprüche,
wobei,
zur Erkennung des abnormalen Ereignisses in der Erkennungseinrichtung Solldaten vorgegeben sind und eine Bewertungseinrichtung vorhanden ist, mit der aus der Erkennungseinrichtung zugeführten, die Herztätigkeit betreffenden Patientendaten das abnormale Ereignis erfassbar ist.

4. Defibrillatorvorrichtung nach Anspruch 3,
wobei
die Solldaten patientenspezifisch vorgebbar sind.

5. Defibrillatorvorrichtung nach einem der vorhergehenden Ansprüche, wobei mittels der Zusatz-Sensoreinrichtung (42) weitere den Patienten und/oder den apparativen Zustand der Defibrillatorvorrichtung betreffende Zusatzinformationen für die Erkennungseinrichtung bereitstellbar sind.

6. Defibrillatorvorrichtung nach einem der vorhergehenden Ansprüche, wobei
die Zusatz-Sensoreinrichtung (42) mindestens einen Lagesensor aufweist, mit dem oder denen die Lage des Körpers feststellbar ist/sind.

7. Defibrillatorvorrichtung nach einem der vorhergehenden Ansprüche, wobei,
der Bewegungssensor und/oder der Lagesensor einen Beschleunigungssensor aufweist/aufweisen.

8. Defibrillatorvorrichtung nach einem der vorhergehenden Ansprüche, wobei
die Zusatz-Sensoreinrichtung (42) eine Dehnmesseinrichtung aufweist.

9. Defibrillatorvorrichtung nach einem der vorhergehenden Ansprüche, wobei
die Zusatz-Sensoreinrichtung (42) Sensoren zum Erfassen des neurologischen Zustandes des Patienten, Sensoren zum Erfassen des Lidschlagreflexes und/oder Sensoren zum Erfassen der Augenbewegung aufweist.

10. Defibrillatorvorrichtung nach einem der vorhergehenden Ansprüche, wobei
eine Sprachausgabe- und/oder Spracheingabeeinheit für Sprachinformation (15) an oder von einer entfernten Kontrollstation vorhanden ist und wobei die Erkennungseinrichtung zur Bewertung von Sprechsignalen des Patienten ausgebildet ist.

11. Defibrillatorvorrichtung nach einem der vorhergehenden Ansprüche, wobei
die Erkennungseinrichtung dazu ausgebildet ist, auf der Grundlage definierter durch den Patienten eingenommener Lage- und/oder Bewegungszustände eine Kalibrierung durchzuführen.

12. Defibrillatorvorrichtung nach einem der Ansprüche 6 bis 11, wobei
die Erkennungseinrichtung so ausgebildet ist, dass die Schockauslösung bei bestimmten Lagen des Patienten priorisiert oder nur bei bestimmten Lagen auslösbar ist.

## Claims

1. External automatic defibrillator with an attachment device for external attachment to and carrying by a patient, with an identification device which is formed to detect an abnormal event of cardiac activity which can be treated by means of electric shock and to analyse movements of the patient, with a defibrillator to apply a shock to the patient after detection of the abnormal event, wherein the identification device can emit an identification signal to the defibrillator which can emit the shock automatically, and with an additional sensor device comprising at least one movement sensor, by means of which a movement of the patient's body can be established which can be detected by means of the identification device, wherein a timer is present which after detecting a ventricular flicker is started for a predetermined time interval of maximum 90 seconds or a minute, wherein the identification device is formed to detect the abnormal event within the time interval, the movement analysis is performed during the time interval, **characterised in that** movements are stored in the defibrillator with which are compared certain movements of the patient which have been previously requested by acoustic, optical or tactile means, and that after expiry of the time interval preparation for application of the shock takes place if no movements which have previously been requested from the patient have been clearly detected.

2. Defibrillator according to claim 1, wherein on the movement analysis a distinction is made between passive and active movement.

3. Defibrillator according to any of the preceding claims, wherein to detect the abnormal event nominal data are provided in the identification device and an assessment device is present which can detect the abnormal event from the patient data concerning the cardiac activity supplied from the identification device.

4. Defibrillator according to claim 3, wherein the nominal data can be prespecified to be specific to the patient.

5. Defibrillator according to any of the preceding claims, wherein by means of the additional sensor device (42) further additional information concerning the patient and/or the apparatus state of the defibrillator can be provided for the identification device.

6. Defibrillator according to any of the preceding claims, wherein the additional sensor device (42) comprises at least one position sensor with which the position of the body can be established.

7. Defibrillator according to any of the preceding claims, wherein the movement sensor and/or the position sensor comprise an acceleration sensor.

8. Defibrillator according to any of the preceding claims, wherein the additional sensor device (42) comprises a strain measurement device.

9. Defibrillator according to any of the preceding claims, wherein the additional sensor device (42) comprises sensors for detecting the neurological state of the patient, sensors for detecting the blink reflex and/or sensors for detecting eye movement.

10. Defibrillator according to any of the preceding claims, wherein a voice output and/or voice input unit is provided for voice information (15) to or from a remote control station and wherein the identification device is formed to evaluate speech signals from a patient.

11. Defibrillator according to any of the preceding claims, wherein the identification device is designed to perform a calibration on the basis of defined position and/or movement states assumed by the patient.

12. Defibrillator according to any of claims 6 to 11, wherein the identification device is formed so that the shock trigger is prioritised in certain positions of the patient or can only be triggered on certain positions.

## Revendications

1. Défibrillateur automatique externe avec un dispositif de fixation pour l'application extérieure et le port dudit défibrillateur sur un patient, avec un dispositif d'identification pour détecter un événement anormal de l'activité cardiaque pouvant être traité au moyen d'un choc électrique et avec un dispositif de défibrillation pour soumettre le patient à un choc après détection de l'événement anormal, un signal d'identification pouvant être délivré par le dispositif d'identification au dispositif de défibrillation et le choc pouvant être délivré automatiquement par ce dernier, et avec un dispositif de détection supplémentaire qui présente au moins un capteur de mouvement au moyen duquel un mouvement du corps du patient peut être constaté, lequel peut être identifié au moyen du dispositif d'identification, un instrument de mesure du temps étant présent qui, après identification d'une fibrillation ventriculaire, est démarré pendant un intervalle de temps prédéterminé d'au plus 90 secondes ou une minute, le dispositif d'identification étant conçu pour détecter l'événement anormal dans l'intervalle de temps, l'analyse de mouvements étant effectuée pendant l'intervalle de temps,
**caractérisé en ce**
**que** dans le défibrillateur sont enregistrés des mouvements avec lesquels sont comparés certains mouvements du patient que celui-ci est préalablement invité à effectuer par voie acoustique, optique ou tactile, et qu'à l'expiration de l'intervalle de temps il est passé à la préparation de l'application du choc si aucun des mouvements que le patient a été préalablement invité à effectuer n'a été clairement identifié.

2. Défibrillateur selon la revendication 1,
dans lequel
il est fait une distinction entre mouvement passif et actif lors de l'analyse de mouvements.

3. Défibrillateur selon une des revendications précédentes,
dans lequel
des données de consigne sont prédéfinies dans le dispositif d'identification pour identifier l'événement anormal et un dispositif d'évaluation est présent, au moyen duquel l'événement anormal peut être détecté à partir de données du patient concernent l'activité cardiaque qui sont amenées au dispositif d'identification.

4. Défibrillateur selon la revendication 3,
dans lequel
les données de consigne peuvent être prédéfinies de manière spécifique à chaque patient.

5. Défibrillateur selon une des revendications précédentes,
dans lequel
des informations supplémentaires concernant le patient et/ou l'état technique du défibrillateur peuvent être mises à disposition pour le dispositif d'identification au moyen du dispositif de détection supplémentaire (42).

6. Défibrillateur selon une des revendications précédentes,
dans lequel
le dispositif de détection supplémentaire (42) présente au moins un capteur de position au moyen duquel ou desquels la position du corps peut être constatée.

7. Défibrillateur selon une des revendications précédentes,
dans lequel
le capteur de mouvement et/ou le capteur de position présente/présentent un capteur d'accélération.

8. Défibrillateur selon une des revendications précédentes,
dans lequel
le dispositif de détection supplémentaire (42) présente un dispositif extensométrique.

9. Défibrillateur selon une des revendications précédentes,
dans lequel
le dispositif de détection supplémentaire (42) présente des capteurs pour détecter l'état neurologique du patient, des capteurs pour détecter le réflexe de clignement des paupières et/ou des capteurs pour détecter le mouvement des yeux.

10. Défibrillateur selon une des revendications précédentes,
dans lequel
une unité de sortie et/ou d'entrée vocale pour une information vocale (15) à destination ou en provenance d'un poste de contrôle éloigné est présente, le dispositif d'identification étant conçu pour évaluer des signaux vocaux du patient.

11. Défibrillateur selon une des revendications précédentes,
dans lequel
le dispositif d'identification est conçu pour effectuer un étalonnage sur la base d'états de position et/ou de mouvement définis pris par le patient.

12. Défibrillateur selon une des revendications 6 à 11,
dans lequel
le dispositif d'identification est conçu de façon que le déclenchement du choc soit rendu prioritaire dans certaines positions du patient ou que le choc ne soit déclenchable que dans certaines positions.
